(19) **Europäisches Patentamt European Patent Office Office européen des brevets**

(11) **EP 1 455 643 B1**

(12) **EUROPEAN PATENT SPECIFICATION**

(45) Date of publication and mention of the grant of the patent:
**25.06.2008 Bulletin 2008/26**

(51) Int Cl.:
**A61B 5/0275** (2006.01) **G01F 1/704** (2006.01)

(86) International application number:
**PCT/DK2002/000889**

(21) Application number: **02790274.1**

(22) Date of filing: **20.12.2002**

(87) International publication number:
**WO 2003/055385 (10.07.2003 Gazette 2003/28)**

(54) **MULTIFIBRE SENSOR FOR MEASURING PERFUSION**

MULTIFASER SENSOR ZUM MESSEN DER DURCHBLUTUNG

CAPTEUR MULTIFIBRE CON U POUR MESURER UNE PERFUSION

(84) Designated Contracting States:
**AT BE BG CH CY CZ DE DK EE ES FI FR GB GR IE IT LI LU MC NL PT SE SI SK TR**

(30) Priority: **21.12.2001 DK 200101950**

(43) Date of publication of application:
**15.09.2004 Bulletin 2004/38**

(73) Proprietor: **Unisense A/S 8000 Århus (DK)**

(72) Inventors:
• **KJAER, Thomas DK-2750 Ballerup (DK)**
• **DAMGAARD, Lars, Riis DK-8000 Aarhus C (DK)**
• **GUNDERSEN, Jens, Kristian DK-8260 Viby (DK)**

• **LARSEN, Lars, Hauer DK-8382 Hinnerup (DK)**

(74) Representative: **Klinge, Ulla Callesen Chas. Hude, 33, H.C. Andersens Boulevard 1780 Copenhagen V (DK)**

(56) References cited:
**WO-A-01/24692        WO-A-97/46853
GB-A- 1 461 345        US-A- 4 274 417**

• **LARSEN O A ET AL: "Blood flow through human adipose tissue determined with radioactive xenon." ACTA PHYSIOLOGICA SCANDINAVICA. SWEDEN MAR 1966, vol. 66, no. 3, March 1966 (1966-03), pages 337-345, XP002236278 ISSN: 0001-6772**

**Description**

Technical Field

The present invention relates to a method for measuring perfusion in tissue, by which method a diffusionally permeable reservoir comprising a tracer is placed on the surface of the tissue, a diffusionally permeable detector chamber for detection of the tracer is placed on the surface of the tissue in a distance from the reservoir, tracer is supplied to the reservoir, and the presence of tracer in the detection chamber is measured. The invention further relates to a sensor for measuring tissue perfusion comprising a diffusionally permeable reservoir and at least two diffusionally permeable detector chambers.

Background Art

Tissue perfusion is a measure of the volume of blood that passes through an amount of tissue per time unit and is often measured in the unit milliliter blood/(100 gram tissue)/minute. Supply of nutrients and excretion of waste products in all tissue take place by diffusion between the tissue cells and the blood, and perfusion in a tissue is therefore an extremely important factor in the health and survival of a tissue. A method for measuring the perfusion of a tissue is therefore very relevant for instance for monitoring tissue during and after surgery and transplantation. Also monitoring of threatened tissue, for instance muscle tissue where the blood supply is threatened by increasing pressure inside the fascia of the muscle, is very relevant as an indicator of when a pressure-releasing operation should be initiated. Likewise, monitoring of the internal perfusion, which is caused by the formation of oedema in the heart, e.g. when the heart has been stopped during surgery, can give important information regarding the need for artificial nutrition for the heart tissue. Furthermore, perfusion is an important parameter in medical research.

A method for determining the perfusion of a tissue using $^{133}$Xenon is described by Larsen, O. A., N. A. Lassen, and F. Quaade. 1966 (Blood Flow through Human Adipose Tissue Determined with Radioactive Xenon. Acta physiol. scand. 66:337-345). The method comprises injection of $^{133}$Xe in the tissue to be investigated and measuring the disappearance rate of the radioactivity. With this technique the temporal resolution is approximately ½ hour, which is inadequate in many acute situations, and the place of the injection is not very precisely defined relative to where the radioactivity is measured.

Another method for measuring perfusion consists of continuous infusion of ethanol during micro dialysis. In micro dialysis a fluid is pumped very slowly through a fiber placed in the tissue of the patient. The ethanol concentration of the fluid in the fiber equilibrates with the surrounding tissue as the fiber is diffusionally permeable and the fluid is collected through a return fiber. Also this method has a very long temporal resolution.

There are patented optical and acoustic techniques (e.g. Laser-Doppler) that measure the movement of blood cells. These methods do not measure the transport of compounds between the tissue cells and the bloodstream but only the velocity of the blood cells. Furthermore the geometry of the measurements is limited to either very local measurements or to measurements in a very limited depth below the surface of a tissue. Other optical techniques include NMR imaging, which require that the patient is immobilized relative to the NMR-scanner.

WO 97/46853 describes a method and a microsensor, which is capable of measuring tissue perfusion, but the measurements of this microsensor are confined to a very limited spatial localization, and heterogeneities in the tissue will make average measurements of the perfusion very complicated. A precise placement and fixation of the microsensor is necessary, which makes it very difficult to use the method on living organisms.

WO 01/24692 describes a method and a sensor for measuring tissue perfusion using tracers that are simultaneously released from and detected by the sensor. This sensor comprises one diffusionally permeable reservoir for releasing the tracer and one diffusionally permeable detector chamber, which makes the response time relatively long and which gives a very complicated signal evaluation.

Disclosure of Invention

To avoid the disadvantages of the prior art for measuring tissue perfusion the purpose of the present invention is to provide a method, which is faster and more precise than the prior art for measuring tissue perfusion.

The purpose is furthermore to provide a sensor, the physical construction of which enables the application on tissue of patients that are not fully immobilized.

Furthermore, it is a purpose to provide a sensor to be used for measurements of skin perfusion through the surface of the skin or perfusion in other organs through the surface of these.

Furthermore, methods will be described that enable the interpretation of the signals of such a sensor with the purpose of calculating the tissue perfusion based on the signals.

Compared to the previously mentioned patent application WO 01/24692 it is possible with the present invention to provide a complete picture of the tracer distribution in the tissue, which can be used to make a relatively easy perfusion rate calculation using only a blood tissue partition coefficient as will be explained below.

In a first aspect, the present invention relates to a method for measuring tissue perfusion by which method a diffusionally permeable reservoir containing a tracer is placed on the surface of a tissue, a diffusionally permeable detector chamber for the tracer is placed on the surface of a tissue in a distance from the reservoir, tracer is supplied to the reservoir, and the presence of tracer in the detector chamber is measured, in which method at least one additional diffusionally permeable detector chamber for detecting the tracer is placed all on the surface of the tissue, and that the at least two detector chambers are located contiguously at different distances from the reservoir, that the presence of tracer is measured in each detection chamber separately, and that the measurements are converted to a measure of the tissue perfusion.

In a second aspect, the present invention relates to a sensor for measuring tissue perfusion comprising a diffusionally permeable reservoir and at least two diffusionally permeable detector chambers, wherein at least two diffusionally permeable detector chambers are located contiguously and in different distances from the reservoir, and that the sensor comprises a detection device for separate measurement of the presence of tracer in each detection chamber.

Brief Description of the Drawing(s)

The invention is explained in detail below with reference to the drawing(s), in which

Figure 1 shows a schematic longitudinal section through a first embodiment of the sensor according to the invention.

Figure 2shows a schematic transversal section through the sensor as indicated by the line A-A on Figure 1.

Figure 3 shows a schematic longitudinal section through a second embodiment of the sensor according to the invention.

Figure 4shows a schematic transversal section through the sensor as indicated by the line A-A on Figure 3.

Figure 5shows a schematic transversal section through a third embodiment of the sensor implemented as a plate or sheet for surface measurements.

Figure 6shows a schematic longitudinal section through a third embodiment of the sensor according to the invention where two reservoirs are interconnected.

Best Mode(s) for Carrying out the Invention

The invention is substantially based on the following basic elements: 1) at least one compartment, which in one particular embodiment is a longitudinally elongated compartment, in the following termed "reservoir", with diffusionally permeable side-walls containing at least one detectable substance like a liquid or a gas, in the following termed "tracer", 2) a source for providing the at least one reservoir with tracer(s), 3) a number of compartments, in the following termed "detector chambers", particularly longitudinally elongated compartments, wherein the tracer(s) can be detected after diffusing out of the reservoir and through the adjacent tissue and into the detector chambers.

In the context of the present invention, "diffusionally permeable" is defined as having the property that the tracer molecules in question can penetrate the material by molecular diffusion, in the absence of holes in the material where mass flow can take place.

In general the principle of the sensor according to the invention is based on having at least one source of a tracer, e.g. a gas, supplied to a reservoir, particularly a longitudinally elongated reservoir, having diffusion permeable side walls through which side walls the tracer will diffuse into the surrounding tissue. Some of the tracer molecules will enter into the blood stream and be carried away while other tracer molecules will move by molecular diffusion and reach one of the at least two detection chambers, which detection chambers in a particular embodiment are longitudinally elongated detection chambers, preferably placed parallel to each other and to the reservoir. The reservoir could in one embodiment be placed in the middle of several detection chambers, where two or more detection chambers are located in increasing distance from the reservoir on each side. In one particular embodiment more than 5 to 20 detection chambers are located on each side of the reservoir. Also it is possible to combine more than one reservoir with the above described embodiments in such a way as to form repeated units of reservoirs interspaced by several (e.g. 10-40) detection chambers. In a further embodiment one reservoir is located at one side having two or more detection chambers in increasing distance on the other side.

In one embodiment the sensor comprises at least 4 detector chambers, particularly at least 5, more particularly at least 8, even more particularly at least 10.

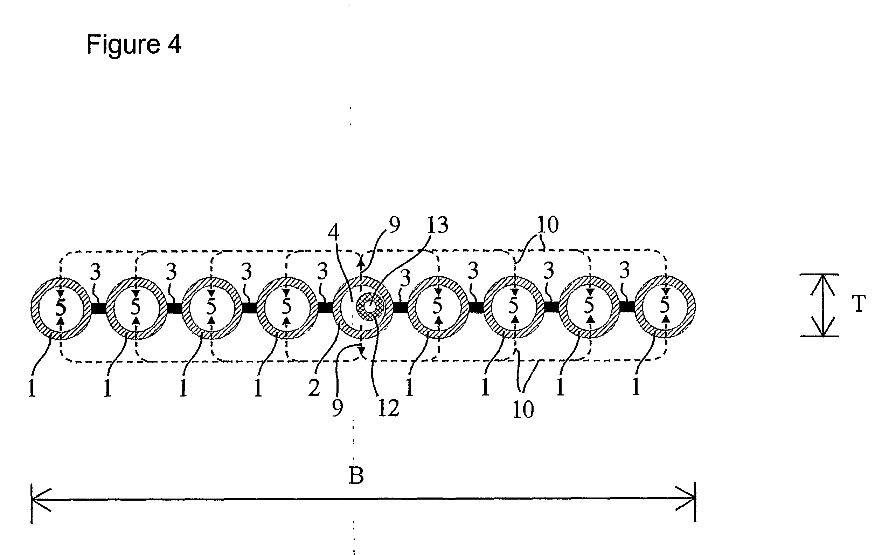

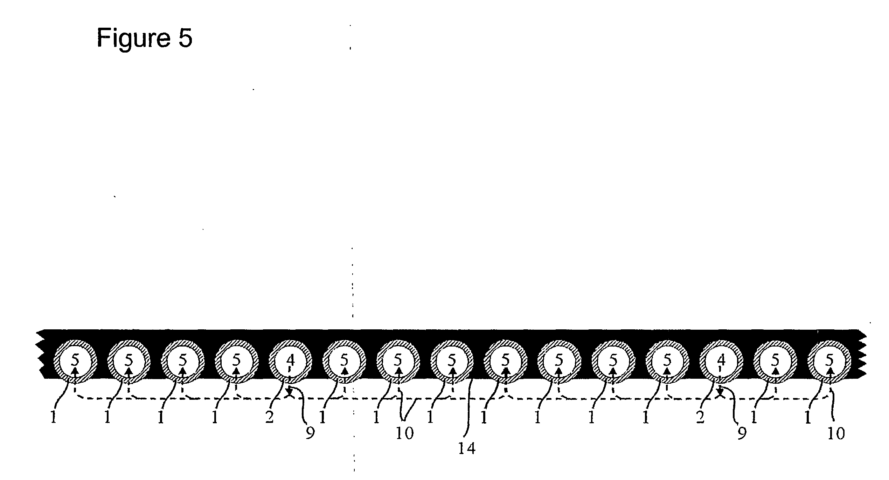

Preferably the at least one reservoir and the detector chambers are fixed in a position parallel to each other. The elongated structures are in one embodiment fixed by tracer-impermeable spacer structures 3, 14 (see Figure 4 and 5).

Alternatively one could imagine other arrangements like e.g. having a central reservoir surrounded by a number of detection chambers forming concentric circles around the reservoir and located in increasing distance from the reservoir.

Many more combinations would of course be imaginable by the skilled person and as long as these possible embodiments enables the detection of the tracer in separate compartments in increasing distance from a given reservoir, such embodiments will fall within the scope of the present invention.

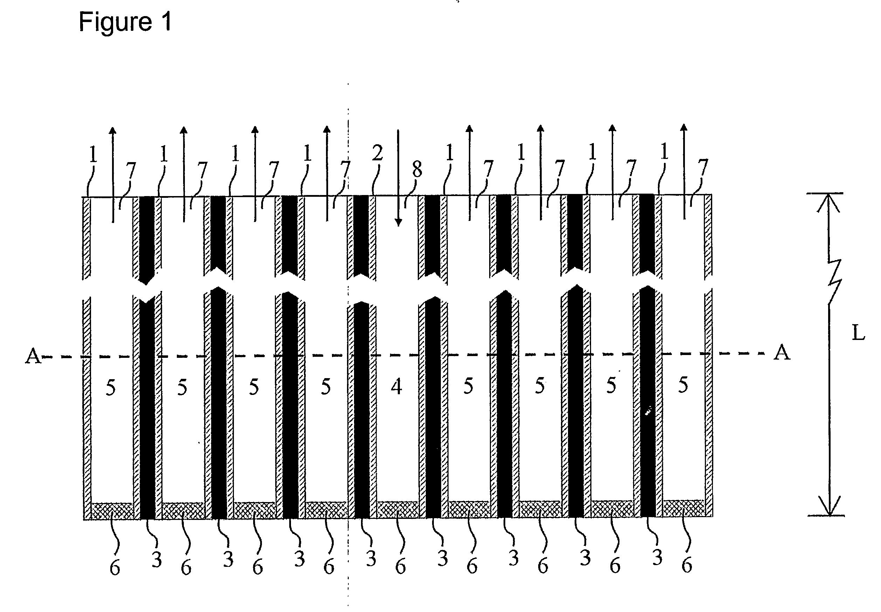

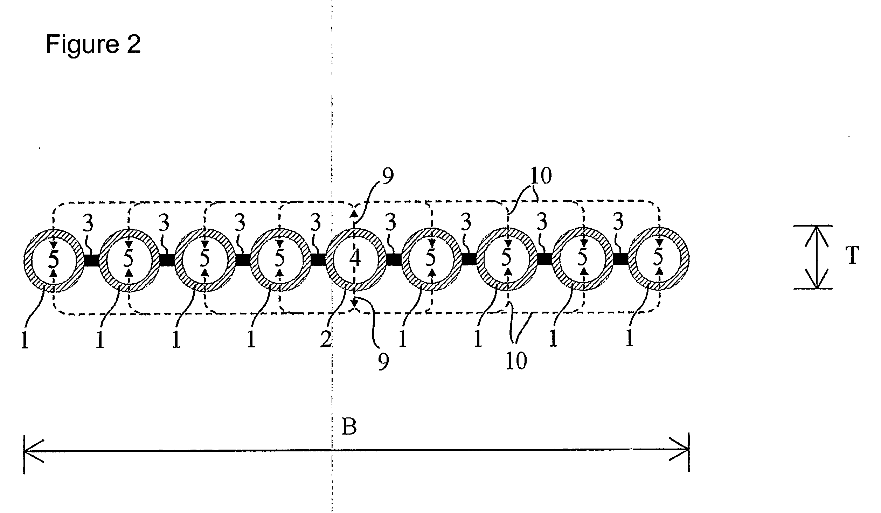

The embodiment of the sensor according to the invention shown in Figure 1 and 2 comprises a reservoir 2 with an inner space 4, which is delimited by a cylindrical reservoir wall, which to a limited degree is diffusionally permeable to tracer molecules. Beside the reservoir 2 a row of detector chambers 1 are placed, which in the shown embodiment have an inner cavity 5. Typically, there are 5-20 detector chambers. Both the reservoir and the detector chambers can be made from e.g. hollow tubes of polymer material such as polyethylene, polypropylene, Teflon, Mylar, Saran, Marprene, Neoprene, butyl-rubber, Tygon, Viton or silicone. Alternatively, the reservoir and detection chambers comprise a relatively impermeable material (thin tubes of metal or metalized polymer, or relatively thick-walled tubes of the above mentioned polymer) which material has pores filled with a more permeable material, for instance silicone polymer. The reservoir and detector chambers are not necessarily made of the same material. For instance, if a very slow tracer is used, a very permeable reservoir should be used to ensure that sufficient tracer enters the tissue. Such a permeable material could be silicone rubber or cellulose acetate. To avoid loss of tissue gases to such a very permeable reservoir, the tracer should be dissolved in liquid, which has been saturated with tissue gases to a physiological level.

Reservoir and detector chambers are connected with connecting spacers 3, which can be produced of e.g. metal ( e.g. silver, gold, steel, cupper) or a relatively impermeable polymer material (such as polyethylene, polypropylene, Teflon, Mylar, Saran, Marprene, Neoprene, butyl rubber, Tygon or Viton), glued between the reservoir and the adj acent detector chambers and between the individual detector chambers. The adhesive material can e.g. be silicone rubber, epoxy resin or acrylic glue. Each detector chamber 1 has an opening 7 in one end connected to a detection device via a tube or pipe. The reservoir 2 has an opening 8 at one end connected to a tracer source, for instance via a thin steel capillary. In the shown embodiment both the reservoir and detector chambers are closed at the opposite ends with a barrier 6, which is primarily impermeable to tracer molecules. The sensor typically has a width B of 0.8-1.2 millimeter, but a width of 0.2-0.8 millimeter can be suitable in tissues with a very high perfusion rate, and a width of 1.2-2.5 millimeter can be suitable in tissues with a very low perfusion rate. The length L is between 5 and 100 millimeter, depending on the size of tissue section that is to be measured and on how sensitive the tissue is to the placement of the sensor. The thickness of the sensor is from 0.02-0.5 millimeters, typically from 0.2-0.5 millimeters, but a thickness of 0.02-0.2 can be more suitable if the tissue is very sensitive to the placement of the sensor.

Instead of separate spacers 3, which form connections between the reservoir 2 and the detector chambers 1, the sensor can be made in one part, for instance by extrusion of a plastic material, e.g. polyethylene or polypropylene, or by photolithography in silicone.

When applying the embodiment shown in Figure 1 and 2, the sensor according to the invention is placed in the tissue which is to be monitored. The placement can be done by fastening a hypodermic needle onto the free end of the sensor and inserting this needle through a section of sufficient length of the tissue, whereby the sensor is pulled into the tissue. Alternatively, a needle which has an open slot along its entire length is used: the sensor is placed in the cavity of the needle through the slot and the needle is inserted into the tissue to be investigated, where after the needle is pulled out, leaving the sensor in the tissue, as the sensor leaves the needle through the slot in the needle. A third way of placing the sensor is to pierce a section of tissue with a hypodermic needle of sufficient size and inserting the sensor or an extension fiber attached to the sensor tip through the tip of the needle so far that the sensor tip or the tip of the extension fiber emerges at the opposite end of the needle. The sensor can be inserted so far that the sensor has reached a position in the needle corresponding to the desired final position in the tissue, before the needle is pulled back out of the tissue or the final positioning of the sensor can be performed after the needle has been withdrawn by pulling one end of the sensor or by pulling an extension fiber attached to the sensor. If the sensor is produced in flexible materials, for instance plastic tubes and silver wires or entirely in plastic material, the sensor can be inserted into the tissue of a patient without causing significant discomfort or injury.

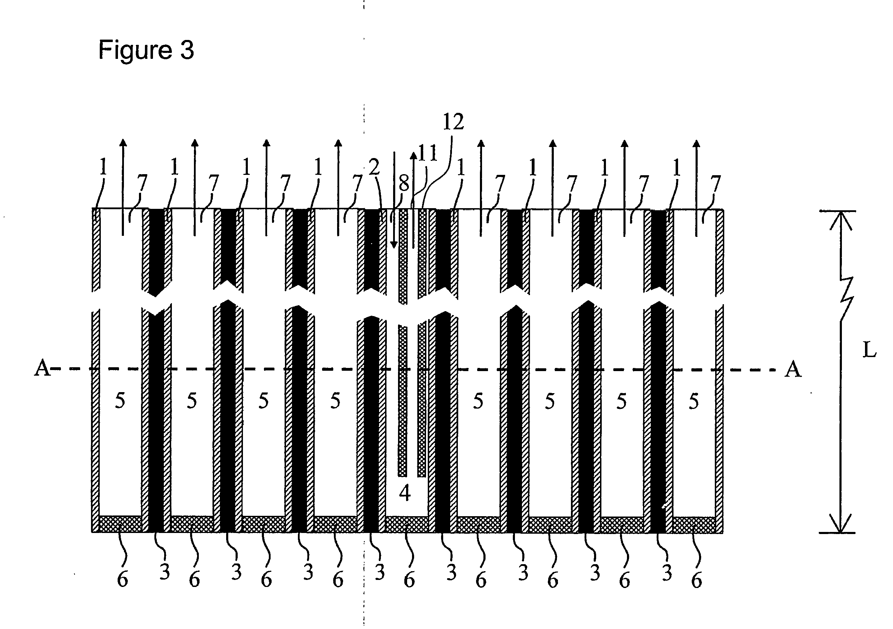

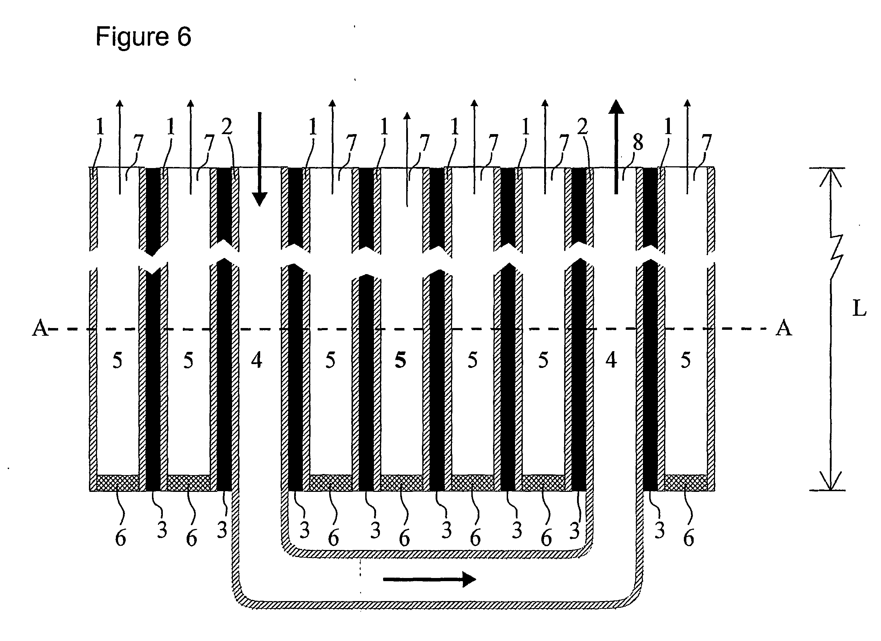

When the sensor is placed in the tissue, tracer(s) in the form of a gas or a liquid is/are supplied in one embodiment in an intermittent fashion to the reservoir. The tracer(s) are supplied through the opening 8 to the reservoir 2 as indicated by the arrow in Figure 1. For gas tracer(s), the tracer(s) can be supplied to the opening 8 and diffuse by molecular diffusion into the entire length of the reservoir. Alternatively, tracer(s) in the form of gas or liquid can be supplied into the reservoir by applying an over-pressure in the tracer source creating a mass flow of tracer into the reservoir. If tracer is supplied as a mass flow in this way, it is advantageous if the tracer can leave the reservoir through an exit opening, as this will allow for a fast exchange of the content of the reservoir. An exit opening can have the form of a return tube 12 with a cavity 13 and an opening 11 as shown in the in embodiment in Figure 3 and 4. The direction of the mass flow is irrelevant, and tracers can therefore be supplied into the reservoir, through the return tube 12 and pass through the reservoir space 4 and leave through the opening 8. The sensor can also have the exit opening implemented so that the reservoir is open in the end opposite to where the tracer(s) enter, i.e. there is no barrier 6. A third way to implement an exit opening is to let the reservoir wherein the tracer flows towards the sensor tip be connected to one or several other reservoirs, in which the tracer(s) flow in the opposite direction. An example of this, where only two reservoirs are connected, is shown in Figure 6.

Tracers that have left the reservoir as part of such a mass flow can either be collected for later disposal or led directly into the atmosphere if the tracer(s) are not harmful.

In one embodiment of the invention only one tracer is supplied to the reservoir and in another embodiment at least two different tracers are supplied to the reservoir.

The tracer(s) will typically consist of a mixture of noble gasses, for instance He, Ne, Ar, Kr, Xe and other non-toxic gasses, for instance $H_2$, $SF_6$ and CFC-gasses. The particular application of the sensor according to the invention determines the choice of tracer gas. The molecular weigh of a gas is negatively correlated to its diffusion coefficient, such that a small molecular weight has a high diffusion coefficient and vice versa. For fast response and overall high signal, a gas having a small molecular weight is selected, however, to achieve a more pronounced signal change in response to perfusion changes, a more heavy molecular weight tracer gas should be selected. If a tracer is very soluble in the tissue components (cells and intercellular material), the signal response to changes in perfusion is less than for a tracer with a low solubility. The solubility of a tracer relies on its polarity. So the tracer(s) should be selected with a polarity that is as different as the general polarity of the tissue components as possible in order to get the strongest signal(s). To take advantage of the improved signal interpretation from two or more signals, a range of tracers that differ significantly with respect to the total effect of molecular weight and polarity on the signal should be selected. Slow diffusing tracer(s) will, if all other parameters are equal, arrive at the detector chamber(s) in smaller amounts than fast tracers. The amount of tracer(s) should reflect the molecular weight such that a slow tracer is represented in larger amounts in the reservoir, to avoid that the resulting signal gets too low to get an acceptable signal-to-noise ratio.

The tracers are in one embodiment supplied to separate reservoirs. In another embodiment the tracers are supplied simultaneously to each reservoir.

When the reservoir 2 contains tracers, part of the tracer molecules will diffuse through the wall of the reservoir 2 and into the surrounding tissue as indicated by the dashed arrows 9 in Figure 2 and 4. In the tissue, a part of these molecules will at some point in time be located inside a blood vessel in the tissue and will be transported away with the blood stream in the vessel. A part of the remaining tracer molecules will diffuse to a detector chamber 1 and diffuse through its wall into the cavity 5, as indicated with the dashed arrows in Figure 2 and 4.

Each of the detector chambers 5 are as mentioned in connection with a detection device via the proximal open end 7. The detection device measures the tracer concentration inside the detection chamber or the amount of tracer that enters the detection chamber per time unit. An example of a technology that measures concentration is optical detection, where a detection device measures the concentration of tracer molecules without consuming these. An example of a technology that measures the amount of tracer that is transported into the detection chamber per time unit is a quadrupol mass spectrometer, which, by keeping a very low pressure inside the mass spectrometer measuring unit, causes a movement of the tracer molecules from the detection chamber of the sensor to the detection mechanism of the mass spectrometer and yields a signal which is proportional to the number of tracer molecules that pass into the measuring chamber per time unit. Another technology that measures the tracer transport by measuring and at the same time removing the tracer is electrochemical oxidation or reduction of the tracer.

In an implementation of the sensor with the simultaneous use of several tracer compounds it is an advantage to use a detection device, which can easily detect several compounds almost simultaneously, for instance the above mentioned quadrupol mass spectrometer, which can measure the signal from several tracers within a few seconds. Each separate detection chamber does not have to have its own detection device. Instead multiplexing can be performed between these chambers, i.e. by using valves or other switching mechanism, the connection between detection chamber and detection device is changed sequentially, such that the detection device measures each detection chamber for a limited time period.

Signal interpretation.

After the detection of tracer gas in each detection chamber, these data have to be converted to a measure for the tissue perfusion. This signal interpretation is in turn dependent on the mode of supply of the tracer(s) to the reservoir (s). Either the supply can be in the form of a sequential or intermittent supply or it can be continuous.

In the case of intermittent supply, in one embodiment the tracer gas is supplied for 1-500 seconds, then the supply of tracer(s) to the reservoir is abruptly interrupted and replaced by a flow of gas or liquid containing none or a different mix of different tracer substances. The length of the supply period is chosen such that within this period and the following detection period only an insignificant part of the tracer has diffused further away from the reservoir than the most distant detection chamber, and this time depends on the type and physiological state of the tissue, the diffusion coefficient of the tracer(s), and the distance between the reservoir and the most remote detection chamber. In practice the end of the supply period is chosen when the tracer has been detected in the detection chamber closest to half way between the reservoir and the detection chamber most distant to the reservoir. The detection period extends from this point to the time when tracer is detected in the most distant detection chamber. After this detection period, there is a resting period until 95-99% of the total tracer amount has been completely removed by diffusion and perfusion, before a new supply period is initiated.

As long as tracer(s) is/are supplied to the reservoir, there will be a net diffusion of tracer(s) in the tissue radially away from the reservoir. The concentration distribution of tracer(s) in the tissue can be monitored by the tracer detection in each detection chamber as mentioned above. The signal from each detection chamber represents the tracer concentration in a distance from the reservoir which is equal to the distance between the reservoir and the detection chamber in question. If the detection chambers are spaced sufficiently finely, the total amount of tracer in the tissue between the reservoir and the outermost detection chamber can thus at any time be approximated as the sum of the concentration detected by each detection chamber times the tissue volume which is represented by the detector chamber. This can be written for equally spaced detection chambers as:

$$Tr_{tot} = \sum C_n \cdot l \cdot \pi \cdot ((r_n + \tfrac{1}{2}dr)^2 - (r_n - \tfrac{1}{2}dr)^2)$$
$$= \sum C_n \cdot l \cdot 2\pi \cdot r \cdot dr$$

where $Tr_{tot}$ is the to total amount of tracer in the tissue, $C_n$ is the concentration at the n'th detection chamber, $r_n$ is the the distance from the reservoir to the n'th detection chamber, $dr$ is the distance between two neighboring detection chambers, and $l$ is the length of the sensor.

When the tracer(s) in the reservoir(s) is abruptly removed and exchanged with none or different tracer(s), the reduction over time of this total amount of tracer in the tissue will be due to the removal by perfusion. If equilibration between the amount dissolved in the cells and interstitial material and the amount dissolved in the blood vessel is assumed, the perfusion can be calculated as:

$$\text{Perfusion} = d(\ln(Tr_{tot}))/dt \cdot \lambda \cdot 100$$

The partition coefficient $\lambda$ has been investigated for a number of gases in different tissues and can be looked up in the literature (e.g. Bülow, J., Jelnes, R., Astrup, A., Madsen, J., and Vilmann, P. 1987. Tissue/blood partition coefficients for xenon in various adipose tissue depots in man. Scand J Clin Lab Invest 47:1-3). When the perfusion is calculated with the above formula with a known partition coefficient for a gas, the partition coefficient of other gases that are used simultaneously can be determined using the same formula for the signal for the gas with unknown partition coefficient, but now with the partition coefficient as the only unknown parameter. In this fashion, the unknown partition coefficient for a gases can be determined and the signal of this gas may be useful for perfusion measurements under conditions for which the gas with the known partition coefficient is not suited, for instance due to substantial changes in the perfusion rate.

Alternatively to the above method with abrupt changes in tracer supply, a continuous tracer supply can be used, but this requires a more complex signal interpretation.

With a continuous supply of tracer(s), the sensor according to the invention indirectly measures the concentration of tracer in the tissue immediately outside each detection chamber by detecting the resulting concentration inside the detection chamber or the resulting transport of tracers into the detection chamber. The concentration of the tracer(s) immediately outside each detection chamber is a function of

1) the tracer concentration inside the reservoir(s)
2) the diffusion coefficient and the solubility of the tracer(s) in the reservoir wall
3) the diffusion coefficient and the solubility of the tracer(s) in surrounding tissue
4) the solubility of the tracer(s) in the blood vessels of the tissue
5) the diffusion coefficient and solubility of the tracer(s) in the wall of the detector chamber must be included in the interpretation of the signals, as there will be a time delay between when the actual changes in the tracer concentration immediately outside the detection chamber occur and when this change is fully reflected by the tracer concentration inside the detection chamber or by the transport of tracer molecules into the detection chamber.
6) the relative volume of blood in blood vessels in the tissue
7) the perfusion (transport of blood in the blood vessels of the tissue), whereby tracer molecules are removed from the tissue.

If the diffusion of a tracer from the reservoir out into a surrounding tissue without perfusion is considered, such diffusion can be described as a cylindrical diffusion system, where the concentration C of the tracer as a function of the diffusion coefficient D, the distance to the reservoir r and the time t can be formulates as:

$$\frac{dC}{dt} = D\frac{1}{r}\frac{d\left(r\frac{dC}{dr}\right)}{dr},$$

A given perfusion, which is evenly distributed in the tissue, will remove a constant fraction, *k*, of the tracer concentration per time unit. If this removal is incorporated into the model, the result will be the following equation:

$$\frac{dC}{dt} = D\frac{1}{r}\frac{d\left(r\frac{dC}{dr}\right)}{dr} - kC$$

This mathematical model assumes that the presence of the detector chambers do not significantly influence the diffusion of the tracer molecules, which can be assumed with a relatively low error margin, provided that the tracer permeability in the wall of the detector chambers is low and if the diffusion coefficient and solubility of a given tracer is the same in the reservoir wall as in the tissue. However, the tissue and the reservoir wall are not likely to have the same solubility and diffusion coefficient for a given tracer as they consist of different materials. For this reason the mathematical model should be expanded to comprise several layers in a cylindrical geometry. This can be done analytically, but such an analytical model cannot handle situations with variable perfusion over time.

It is therefore useful to employ a computer model, which implements the reservoir wall and tissue as a cylindrical diffusion-consumption system. The system is divided into layers and the computer model moves amounts of a tracer between neighboring layers as a function of the concentration difference between them and of the tracer diffusion coefficient and solubility in each layer. The amount moved is determined using a discrete version of the above equation. A similar model system can be implemented for a detection chamber wall, and by combining these two systems, a complete model of the signal is for a detection chamber can be constructed.

To use the model to model the perfusion rate in the tissue, it is necessary to have values for the above-mentioned parameters under 1) - 6). If these values are known, the actual signals can be modeled by an iterative procedure where the perfusion rate is found as the one that gives the model the best fit to the signals. If the values for diffusion coefficients and solubility are not known beforehand, they can also be variable parameters in the model that are adjusted to give the model the best fit to the signals, but with the difference that they can be assumed to be constant during the measurement.

By using two or more tracers simultaneously, the model can be consolidated, as the tracers share the same perfusion rate and the number of unknown parameters per tracer in the model is thus reduced. For instance helium and krypton, that have very different diffusion coefficients, can be used simultaneously.

A simpler way to interpret the signal in the case of continuous tracer supply is a qualitative evaluation. If the perfusion of the tissue increases, the tracer concentration- and thus the sensor signal - will decrease. If the signal is monitored continuously, it will thus be possible to monitor changes in perfusion qualitatively, as all other parameters under 2) to 6) above can be assumed to be constant for each tracer for a given tissue for a given person on a given day.

The sensor can be formed with different geometries and of different materials, such that the sensitivity of the sensor and other properties are suited for different measuring situations. By increasing the distance from the reservoir to the most distant detector chamber the sensitivity of the sensor for low perfusion rates is increased, but this has the disadvantage that the sensor width is increased and thus the risk of discomfort or irritation of the tissue is increased. Conversely, the tissue can be less damaged by using a slimmer version of the sensor, but at the expense of the sensitivity at low perfusion rates. Inner and outer diameter of the reservoir can be increased, whereby the tracer will reach tissue in a larger circumference relative to the sensor and the measurement will thus integrate over a larger tissue section, but this increase is at the expense of the sensitivity at low perfusion rates. The tracer permeability of the walls of both reservoir and detector chambers can be varied by changing material (e.g. increasing the permeability by changing from Teflon to polyethylene, from polyethylene to polypropylene, or from polyethylene to Mylar) or making a different wall thickness. An increase of the permeability will increase the absolute size of the signal and thus the signal-to-noise ratio, but it will also result in that the naturally occurring tissue gases like oxygen and carbon dioxide will diffuse into the reservoir and/or the detector chambers to a higher degree whereby the physiological state of the tissue may be affected.

In Figure 5 a schematic transversal section of an embodiment of the sensor according to the invention is shown, where several reservoirs 2 and detection chambers 1 are laid out side by side on the underside of a tracer-impermeable sheet or plate 14. This plate can be fastened to the surface of a patient's skin or the surface of an organ and according to the principles described above, multiple tracers can diffuse from the reservoirs to multiple detector chambers and an interpretation of the resulting signal will enable a quantification of the perfusion in the skin or the organ. The reservoirs and detector chambers in this arrangement can also be realized by cutting grooves in the underside of a plate and covering them all with a common membrane. The larger the plate, the more reservoirs and detector chambers that can fit on its underside, and the larger the absolute signal and thus signal-to-noise ratio will be. However, the larger the plate, the more difficult it will be to make a tight fit between the plate and a tissue surface, and the larger an area the measurement will integrate over. Thus the size and shape of the plate should be chosen for a specific application as a compromise between desired size of the absolute signal and of the signal-to-noise ratio on one side and the tissue area to be measured on the other. The shape of the plate can be varied for the application (e.g. circular, oval, or rectangular). Typically, the plate is more than 5 millimeter long, more than 5 millimeter wide, and more than 0.3 millimeter thick and less than 100 millimeter long, 100 millimeter wide and less than 10 millimeter thick.

The plate can be fastened to the tissue surface by mechanical means (e.g. straps) or by suction in specialized channels in the underside of the plate.

In addition to tracer molecules, also naturally occurring tissue gases (e.g. oxygen, carbon dioxide) can diffuse into the detector chambers. If the detection device is sensitive to these gases, as is for instance a quadrupol mass spectrometer, these gases can be measured almost simultaneously with the tracer(s) and thus further important information about the state of the tissue is obtained.

Above, several embodiments are described. It is understood that these are only a few of many possible embodiments of the described principle, as it is defined in the accompanying set of claims and that other embodiments can be conceived by a person skilled in the art. For instance, the reservoir and detector chambers in the embodiment in Fig. 5 are shown as parallel tubes, but other geometric configurations, as for instance a series of concentric rings, is also a functional configuration. Likewise, the sensor in the embodiments shown in Figure 1, 2, 3 and 4 are described as a band of parallel tubes, but both reservoir and detector chambers can also be implemented as for instance parallel membrane-covered grooves in a common central cylindrical structure, which is impermeable to tracers, which in principle would correspond to the plate 14 in Figure 5 being a cylindrical structure.

**Claims**

1. A method for measuring perfusion in tissue, by which method a diffusionally permeable reservoir for a tracer is placed on the surface of a tissue, a diffusionally permeable detector chamber for the tracer is placed on the surface of a tissue in a distance from the reservoir, tracer is supplied to the reservoir, and the presence of tracer in the detector chamber is measured, **characterized in that** at least one additional diffusionally permeable detector chamber for detecting the tracer is placed on the surface of the tissue, and that the at least two detector chambers are located contiguously at different distances from the reservoir, that the presence of tracer is measured in each detection chamber separately, and that the measurements are converted to a measure of the tissue perfusion.

2. The method according to claim 1, **characterized in that** at least two detector chambers are located contiguously in increasing distances from the reservoir on both sides of the reservoir.

3. The method according to claims 1 or 2, **characterized in that** at least one additional diffusionally permeable reservoir for a tracer is provided and that tracer is supplied to each reservoir.

4. The method according to claim 3, **characterized in that** the at least two detector chambers are placed contiguously between the reservoir and any additional diffusionally permeable reservoir.

5. The method according to any of the preceding claims, **characterized in that** at least two different tracer compounds are applied.

6. The method according to claim 5, **characterized in that** the at least two different tracer compounds are supplied to separate reservoirs.

7. The method according to claim 5 or 6, **characterized in that** all tracers are supplied simultaneously to each reservoir.

8. The method according to any of the preceding claims, **characterized in that** the tracer(s) is continuously supplied to each reservoir.

9. The method according to any of the claims 1 - 7, **characterized in that** the tracer is sequentially/intermittently supplied and removed from each reservoir in abrupt changes with a time interval of between 1 second and 500 seconds between two consecutive changes.

10. The method according to claim 9, **characterized in that** signal interpretation is performed by evaluating the disappearance of the total amount of tracer after the abrupt removal of tracer from the reservoir.

11. A sensor for measuring tissue perfusion comprising a diffusionally permeable reservoir and at least two diffusionally permeable detector chambers, **characterized in that** at least two diffusionally permeable detector chambers are located contiguously and in different distances from the reservoir, and that the sensor comprises a detection device for separate measurement of the presence of tracer in each detection chamber.

12. The sensor according to claim 11, **characterized in that** at least two difusionally permeable detector chambers are located contiguosly on both sides of the reservoir.

13. The sensor according to claim 11, **characterized in that** it comprises at least one additional diffusionally permeable tracer reservoir located at a distance from the first reservoir.

14. The sensor according to claim 13, **characterized in that** the at least two detector chambers are located contiguously between the reservoir and any additional diffusionally permeable reservoir.

15. The sensor according to claim 11, **characterized in that** the detection device is a mass spectrometer.

16. The sensor according to any of the claims 11-15, **characterized in that** it comprises a valve device for sequentially connecting the detection device to each detection chamber.

17. The sensor according to any of the claims 11-16, **characterized in that** the at least one reservoir and the detector chambers are fixed in a position parallel to each other and are elongated parallel structures with diffusionally permeable walls.

18. The sensor according to any of the claims 11-17, **characterized in that** the reservoir(s) and detection chambers are made of a polymer, such as polyethylene or polypropylene.

19. The sensor according to claim 17, **characterized in that** the elongated structures are fixed by a tracer-impermeable spacer structure (3,14).

20. The sensor according to any of the claims 11-19, **characterized in that** each reservoir and each detector chamber consist of a separate channel in a corn mon structure, for instance an extruded plastic structure or a structure made by photolithography.

21. The sensor according to any of the claims 11-20, **characterized in that** each reservoir and each detector chamber is produced as an elongated chamber, preferably with a length of over 5 millimeter and a maximum inner diameter of less than 1 millimeter, more preferably with a length of more than 10 millimeter and a maximum inner diameter of less than 0.5 millimeter, and even more preferably with a length of over 50 millimeter and a maximum inner diameter of less than 0.2 millimeter.

22. The sensor according to any of the claims 11-21, **characterized in that** tracer is supplied to each reservoir by molecular diffusion through an entrance opening (8) to the reservoir.

23. The sensor according to claim 22, **characterized in that** each reservoir in addition to the entrance opening has an exit opening for the tracer.

24. The sensor according to claim 23, **characterized in that** the exit opening of each reservoir is an opening in an exit tube, which is located inside the reservoir cavity.

25. The sensor according to claims 23 or 24, **characterized in that** the tracer is supplied to each reservoir by mass flow, flowing through the reservoir from the entrance opening to the exit opening.

26. The sensor according to any of the claims 11-16, **characterized in that** each reservoir and each detector chamber is provided as a groove in a sheet or plate, which is tracer-impermeable, and that each groove is covered by a membrane that is diffusionally permeable to tracer molecules.

27. The sensor according to any of the claims 11-16, **characterized in that** each reservoir and each detector chamber is provided as a groove in a cylindric surface of a cylindric structure, which is tracer-impermeable, and that each groove is covered by a membrane that is diffusionally permeable to tracer molecules.

**Patentansprüche**

1. Verfahren zum Messen einer Durchströmung in einem Gewebe, wobei durch dieses Verfahren ein diffusionsbedingt durchlässiges Reservoir für einen Tracer auf der Oberfläche eines Gewebes angeordnet wird, eine diffusionsbedingt durchlässige Detektorkammer für den Tracer auf der Oberfläche eines Gewebes in einem Abstand vom Reservoir angeordnet wird, der Tracer an das Reservoir geliefert wird und das Vorhandensein des Tracers in der Detektorkammer gemessen wird, **dadurch gekennzeichnet, dass** wenigstens eine zusätzliche diffusionsbedingt durchlässige Detektorkammer zum Detektieren des Tracers auf der Oberfläche des Gewebes angeordnet wird, und dass die wenigstens zwei Detektorkammern fortlaufend in unterschiedlichen Abständen vom Reservoir angeordnet sind, dass das Vorhandensein des Tracers in jeder Detektorkammer separat gemessen wird und dass die Messungen in eine Messung der Gewebedurchströmung konvertiert werden.

2. Verfahren nach Anspruch 1, **dadurch gekennzeichnet, dass** wenigstens zwei Detektorkammern auf beiden Seiten des Reservoirs fortlaufend in zunehmenden Abständen vom Reservoir angeordnet werden.

3. Verfahren nach den Ansprüchen 1 oder 2, **dadurch gekennzeichnet, dass** wenigstens ein zusätzliches diffusionsbedingt durchlässiges Reservoir für einen Tracer bereitgestellt wird und dass der Tracer jedem Reservoir zugeführt wird.

4. Verfahren nach Anspruch 3, **dadurch gekennzeichnet, dass** die wenigstens zwei Detektorkammern fortlaufend zwischen dem Reservoir und jedem zusätzlichen diffusionsbedingt durchlässigen Reservoir angeordnet werden.

5. Verfahren nach einem der vorhergehenden Ansprüche, **dadurch gekennzeichnet, dass** wenigstens zwei unterschiedliche Tracerverbinctungen verwendet werden.

6. Verfahren nach Anspruch 5, **dadurch gekennzeichnet, dass** die wenigstens zwei unterschiedlichen Tracerverbindungen den separaten Reservoirs zugeführt werden.

7. Verfahren nach Anspruch 5 oder 6, **dadurch gekennzeichnet, dass** alle Tracer gleichzeitig jedem Reservoir zugeführt werden.

8. Verfahren nach einem der vorhergehenden Ansprüche, **dadurch gekennzeichnet, dass** der/die Tracer jedem Reservoir kontinuierlich zugeführt wird/werden.

9. Verfahren nach einem der Ansprüche 1 bis 7, **dadurch gekennzeichnet, dass** der Tracer jedem Reservoir sequentiell/schubweise in abrupten Änderungen mit einem Zeitintervall von zwischen 1 Sekunden und 500 Sekunden zwischen zwei aufeinanderfolgenden Änderungen zugeführt und von diesem entnommen wird.

10. Verfahren nach Anspruch 9, **dadurch gekennzeichnet, dass** die Signalinterpretation durchgeführt wird, indem das Verschwinden der gesamten Tracermenge nach der abrupten Entnahme des Tracers aus dem Reservoir bewertet wird.

11. Sensor zum Messen einer Gewebedurchströmung mit einem diffusionshedingt durchlässigen Reservoir und wenigstens zwei diffusionsbedingt durchlässigen Detektorkarmmern, **dadurch gekennzeichnet, dass** wenigstens zwei diffusionsbedingt durchlässige Detektorkammern fortlaufend und in unterschiedlichen Abständen vom Reservoir angeordnet sind und dass der Sensor eine Detektionseinrichtung für die separate Messung des Vorhandenseins des Tracers in jeder Detektionskammer umfasst.

12. Sensor nach Anspruch 11, **dadurch gekennzeichnet, dass** wenigstens zwei diffusionsbedingt durchlässige Detektorkammern fortlaufend auf beiden Seiten des Reservoirs angeordnet sind.

13. Sensor nach Anspruch 11, **dadurch gekennzeichnet, dass** dieser wenigstens ein zusätzliches diffusionsbedingt durchlässiges Tracerreservoir umfasst, das in einem Abstand von dem ersten Reservoir angeordnet ist.

14. Sensor nach Anspruch 13, **dadurch gekennzeichnet, dass** die wenigstens zwei Detektorkammern fortlaufend zwischen dem Reservoir und jedem zusätzlichen diffusionsbedingt durchlässigen Reservoir angeordnet sind.

15. Sensor nach Anspruch 11, **dadurch gekennzeichnet, dass** die Detektionseinrichtung ein Massenspektromcter ist.

16. Sensor nach einem der Ansprüche 11 bis 15, **dadurch gekennzeichnet, dass** dieser eine Ventileinrichtung zum sequentiellen Verbinden der Detektionseinrichtung mit jeder Detektionskammer umfasst.

17. Sensor nach einem der Ansprüche 11 bis 16, **dadurch gekennzeichnet, dass** das wenigstens eine Reservoir und die Detektorkammern in einer Position parallel zueinander fixiert sind und längliche parallele Strukturen mit zur Diffusion durchlässigen Wänden sind.

18. Sensor nach einem der Ansprüche 11 bis 17, **dadurch gekennzeichnet, dass** das/die Reservoir(e) und Detektionskammcrn aus einem Polymer hergestellt sind, wie Polyethylen oder Polypropylen.

19. Sensor nach Anspruch 17, **dadurch gekennzeichnet, dass** die länglichen Strukturen durch eine für den Traccr undurchlässige Abstandsstruktur (3, 14) fixiert sind.

20. Sensor nach einem Ansprüche 11 bis 19, **dadurch gekennzeichnet, dass** jedes Reservoir und jede Detektorkammer aus einem separaten Kanal in einer gemeinsamen Struktur besteht, zum Beispiel einer extrudierten Kunststoffstruktur oder einer Struktur, die durch Fotolithografie hergestellt ist.

21. Sensor nach einem der Ansprüche 11 bis 20, **dadurch gekennzeichnet, dass** jedes Reservoir und jede Detektorkammer als eine längliche Kammer hergestellt ist, vorzugsweise mit einer Länge von über 5 Millimeter und einem maximalen Innendurchmesser von weniger als 1 Millimeter, vorzugsweise mit einer Länge von mehr als 10 Millimeter und einem maximalen Innendurchmesser von weniger als 0,5 Millimeter und noch bevorzugter mit einer Länge von über 50 Millimeter und einem maximalen Innendurchmesser von weniger als 0,2 Millimeter.

22. Sensor nach einem der Ansprüche 11 bis 21, **dadurch gekennzeichnet, dass** der Tracer jedem Reservoir durch eine Molekulardiffusion durch eine Eingangsöffnung (8) in das Reservoir zugeführt wird.

23. Sensor nach Anspruch 22, **dadurch gekennzeichnet, dass** jedes Reservoir zusätzlich zu der Eingangsöffnung eine Ausgangsöffnung für den Tracer hat.

24. Sensor nach Anspruch 23, **dadurch gekennzeichnet, dass** dic Ausgangsöffnung jcdcs Reservoirs eine Öffnung in ein Ausgangsrohr ist, welches innerhalb des Rcservoirraumes angeordnet ist.

25. Sensor nach den Ansprüchen 23 oder 24, **dadurch gekennzeichnet, dass** der Tracer jedem Reservoir durch einen Massenfluss zugeführt wird, der durch das Reservoir von der Eingangsöffnung zur Ausgangsöffnung hindurchfließt.

26. Sensor nach einem dcr Ansprüche 11 bis 16, **dadurch gekennzeichnet, dass** jedes Reservoir und jede Detektorkammer als eine Rinne in einem Flächengebilde oder in einer Platte vorgesehen ist, welches/welche für den Tracer undurchlässig ist und dass jede Rinne durch eine Membrane abgedeckt ist, die für Tracermoleküle diffusionsbedingt durchlässig ist.

27. Sensor nach einem der Ansprüche 11 bis 16, **dadurch gekennzeichnet, dass** jedes Reservoir und jede Detektorkammer als eine Rinne in einer zylindrischen Oberfläche einer Zylinderstruktur vorgesehen ist, die für den Tracer undurchlässig ist, und dass jede Rinne durch eine Membrane abgedeckt ist, die für die Tracermoleküle diffusionsbedingt durchlässig ist.

**Revendications**

1. Procédé pour mesurer une perfusion d'un tissu, procédé dans lequel un réservoir perméable par diffusion pour une traceur est placé sur la surface d'un tissu, une chambre de détecteur perméable par diffusion pour le traceur est placée sur la surface d'un tissu à distance du réservoir, le traceur est délivré au réservoir, et la présence du traceur dans la chambre de détecteur est mesurée, **caractérisé en ce qu'**au moins une chambre de détecteur perméable par diffusion supplémentaire pour détecter le traceur est placée sur la surface du tissu, et **en ce qu'**au moins deux chambres de détecteur sont placées de façon contiguë à des distances différentes du réservoir, **en ce que** la présence du traceur est mesurée dans chaque chambre de détection séparément, et **en ce que** les mesures sont converties en une mesure de la perfusion d'un tissu.

2. Procédé selon la revendication 1, **caractérisé en ce qu'**au moins deux chambres de détecteur sont situées de façon contiguë à distance croissante du réservoir sur les deux côtés du réservoir.

3. Procédé selon la revendication 1 ou 2, **caractérisé en ce qu'**au moins un réservoir perméable par diffusion supplémentaire pour un traceur est prévu et **en ce qu'**un traceur est délivré à chaque réservoir.

4. Procédé selon la revendication 3, **caractérisé en ce qu'**au moins deux chambres de détecteur sont placées de façon contiguë entre le réservoir et un autre réservoir perméable par diffusion supplémentaire.

5. Procédé selon l'une quelconque des revendications précédentes, **caractérisé en ce qu'**au moins deux composés différents de traceur sont appliqués.

6. Procédé selon la revendication 5, **caractérisé en ce que** les au moins deux composés différents de traceur sont délivrés à des réservoirs distincts.

7. Procédé selon la revendication 5 ou 6, **caractérisé en ce que** tous les traceurs sont délivrés simultanément à chaque réservoir.

8. Procédé selon l'une quelconque des revendications précédentes, **caractérisé en ce que** le traceur (les traceurs) est(sont) délivré(s) de façon continue à chaque réservoir.

9. Procédé selon l'une quelconque des revendications 1-7, **caractérisé en ce que** le traceur est délivré séquentiellement/par intermittence et éliminé de chaque réservoir lors de changements brusques avec un intervalle de temps compris entre 1 seconde et 500 secondes entre deux changements consécutifs.

10. Procédé selon la revendication 9, **caractérisé en ce que** l'interprétation du signal est effectuée en évaluant la disparition de la quantité totale de traceur après la brusque élimination du traceur du réservoir.

11. Capteur pour mesurer une perfusion de tissu comportant un réservoir perméable par diffusion et au moins deux chambres de détecteur perméables par diffusion, **caractérisé en ce qu'**au moins deux chambres de détecteur perméables par diffusion sont situées de façon contiguë et à différentes distances du réservoir, et **en ce que** le capteur comporte un dispositif de détection pour la mesure séparée de la présence du traceur dans chaque chambre de détection.

12. Capteur selon la revendication 11, **caractérisé en ce qu'**au moins deux chambres de détecteur perméables par diffusion sont situées de façon contiguë sur les deux côtés du réservoir.

13. Capteur selon la revendication 11, **caractérisé en ce qu'il** comporte au moins un réservoir de traceur perméable par diffusion supplémentaire situé à distance du premier réservoir.

14. Capteur selon la revendication 13, **caractérisé en ce que** les au moins deux chambres de détecteur sont situées de façon contiguë entre le réservoir et un réservoir perméable par diffusion supplémentaire.

15. Capteur selon la revendication 11, **caractérisé en ce que** le dispositif de détection est un spectromètre de masse.

**16.** Capteur selon l'une quelconque des revendications 11-15, **caractérisé en ce qu'**il comporte un dispositif de soupape pour relier séquentiellement le dispositif de détection à chaque chambre de détection.

**17.** Capteur selon l'une quelconque des revendications 11-16, **caractérisé en ce que** le au moins un réservoir et les chambres de détecteur sont fixés dans une position parallèle les uns aux autres et sont des structures parallèles allongées avec des parois perméables par diffusion.

**18.** Capteur selon l'une quelconque des revendications 11-17, **caractérisé en ce que** le(s) réservoir(s) et les chambres de détection sont réalisés en un polymère, tel que le polyéthylène ou le polypropylène.

**19.** Capteur selon la revendication 17, **caractérisé en ce que** les structures allongées sont fixées par une structure d'espacement imperméable au traceur (3, 14).

**20.** Capteur selon l'une quelconque des revendications 11-19, **caractérisé en ce que** chaque réservoir et chaque chambre de détecteur consistent en un canal séparé dans une structure en forme de lune, par exemple une structure en plastique extrudé ou une structure réalisée par photolithographie.

**21.** Capteur selon l'une quelconque des revendications 11-20, **caractérisé en ce que** chaque réservoir et chaque chambre de détecteur sont fabriqués sous la forme d'une chambre allongée, de préférence avec une longueur supérieure à 5 mm et un diamètre interne maximum inférieur à 1 mm, de préférence avec une longueur supérieure à 10 mm et un diamètre interne maximum inférieur à 0,5 mm, et de façon plus préférable encore avec une longueur supérieure à 50 mm et un diamètre interne maximum inférieur à 0,2 mm.

**22.** Capteur selon l'une quelconque des revendications 11-21, **caractérisé en ce que** le traceur est délivré à chaque réservoir par diffusion moléculaire par l'intermédiaire d'un orifice d'entrée (8) au réservoir.

**23.** Capteur selon la revendication 22, **caractérisé en ce que** chaque réservoir, en plus de l'orifice d'entrée, possède un orifice de sortie pour le traceur.

**24.** Capteur selon la revendication 23, **caractérisé en ce que** l'orifice de sortie de chaque réservoir est un orifice dans un tube de sortie, qui est situé à l'intérieur de la cavité du réservoir.

**25.** Capteur selon les revendications 23 ou 24, **caractérisé en ce que** le traceur est délivré à chaque réservoir par débit massique, s'écoulant à travers le réservoir depuis l'orifice d'entrée vers l'orifice de sortie.

**26.** Capteur selon l'une des revendications 11-16, **caractérisé en ce que** chaque réservoir et chaque chambre de détecteur sont réalisés sous la forme d'une gorge dans une feuille ou une plaque, qui est imperméable au traceur, et **en ce que** chaque gorge est recouverte par une membrane qui est perméable par diffusion aux molécules du traceur.

**27.** Capteur selon l'une des revendications 11-16, **caractérisé en ce que** chaque réservoir et chaque chambre de détecteur sont réalisés sous la forme d'une gorge dans une surface cylindrique d'une structure cylindrique, qui est imperméable au traceur, et **en ce que** chaque gorge est recouverte par une membrane qui est perméable par diffusion aux molécules du traceur,

Figure 1

EP 1 455 643 B1

Figure 2

EP 1 455 643 B1

Figure 3

Figure 4

Figure 5

EP 1 455 643 B1

Figure 6

EP 1 455 643 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9746853 A

- WO 0124692 A

**Non-patent literature cited in the description**

- **LARSEN, O. A. ; N. A. LASSEN ; F. QUAADE.** Blood Flow through Human Adipose Tissue Determined with Radioactive Xenon. *Acta physiol. scand.,* 1966, vol. 66, 337-345

- **BÜLOW, J. ; JELNES, R. ; ASTRUP, A. ; MADSEN, J. ; VILMANN, P.** Tissue/blood partition coefficients for xenon in various adipose tissue depots in man. *Scand J Clin Lab Invest,* 1987, vol. 47, 1-3